(19) Europäisches Patentamt
European Patent Office
Office européen des brevets

(11) EP 0 743 297 B1

(12) **EUROPÄISCHE PATENTSCHRIFT**

(45) Veröffentlichungstag und Bekanntmachung des Hinweises auf die Patenterteilung:
**19.01.2000 Patentblatt 2000/03**

(51) Int Cl.[7]: **C07C 49/553**, C07C 49/613, C07C 49/543, C07C 49/603, C11B 9/00, A61K 7/46

(21) Anmeldenummer: **96107506.6**

(22) Anmeldetag: **10.05.1996**

(54) **Neue Riechstoffe**

Novel perfume

Parfum nouveau

(84) Benannte Vertragsstaaten:
**CH DE ES FR GB IT LI NL**

(30) Priorität: **16.05.1995 CH 141795**

(43) Veröffentlichungstag der Anmeldung:
**20.11.1996 Patentblatt 1996/47**

(73) Patentinhaber: **GIVAUDAN-ROURE (INTERNATIONAL) S.A.**
**1214 Vernier, Genève (CH)**

(72) Erfinder:
- **Bajgrowicz, Jerzy A.**
  **8053 Zürich (CH)**
- **Bringhen, Alain**
  **1257 Croix-de-Rozon (CH)**
- **Frater, Georg**
  **8404 Winterthur (CH)**
- **Müller, Urs**
  **8051 Zürich (CH)**

(74) Vertreter: **Buntz, Gerhard et al**
**GIVAUDAN-ROURE (INTERNATIONAL) SA,**
**Postfach 3255**
**4002 Basel (CH)**

(56) Entgegenhaltungen:
**EP-A- 0 464 357** **US-A- 3 929 677**

- **CHEMICAL ABSTRACTS, vol. 54, no. 3, 10.Februar 1960 Columbus, Ohio, US; abstract no. 2402, MOUSSERON-CANET ET AL.: "Novel Syntheses in the 1,1,8-trimethyl-octahydonaphthalene series" XP002010759 & BULL. SOC. CHIM. FRANCE, 1959, Seite 601-606 MOUSSERON-CANET ET AL.: "Sur quelques composés diéniques conjugués dérivés du citral."**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue Riechstoffe. Es handelt sich dabei um die Verbindungen der Formel

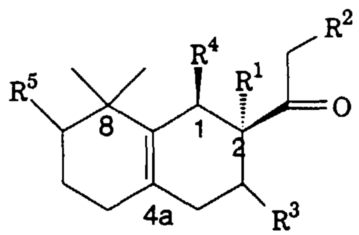

I

worin

$$R^1 = R^4 = CH_3 \text{ und } R^2 = R^3 = H$$

oder

$$R^1 = R^2 = R^3 = H \text{ und } R^4 = CH_3,$$

oder

$$R^1 + R^2 = -CH_2CH_2-$$

und

$$R^3 = H \text{ und } R^4 = CH_3,$$

oder

$$R^1 = R^4 = CH_3 \text{ und } R^2 + R^3 = -CH_2-$$

oder

$$R^1 = CH_3 \text{ und } R^2 = R^3 = H$$

und

$$R^4 = CH_2CH_3$$

oder

$$R^1 = R^2 = R^4 = CH_3$$

und

$$R^3 = H$$

sind, und

$$R^5 = H$$

(bevorzugt) oder $CH_3$ ist.

Die Formel I soll auch die möglichen Enantiomeren als auch das Racemat im Falle von $R^3 = R^5 = H$ und die Diastereomeren im Falle von Substituenten $R^3$ und $R^5$ umfassen.

Die Erfindung betrifft auch Riechstoffkompositionen mit einem Gehalt an I, sowie die Verwendung von I als Riechstoffe.

Aus EP 464 357, Beispiel 5, ist u.a. die Verbindung der Formel

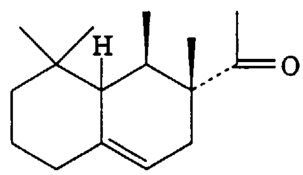

VI

als Riechstoff bekannt geworden.

Verschieben der Doppelbindung Δ 4,4a zu Δ 8a,4a in dieser Verbindung VI, z.B. durch saure Isomerisierung, führt zu der praktisch geruchlosen Verbindung, dem Diastereomeren der Formel

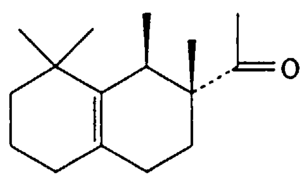

VII

Aenderung der Stereochemie in $C_1$ oder $C_2$ führt überraschenderweise zu einer neuen, äussert geruchsstarken Verbindung der Formel

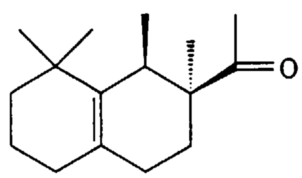

I'

mit interessanten Riechstoffeigenschaften.

Solche Geruchseigensschaften kommen nun ganz allgemein den Verbindungen der Formel

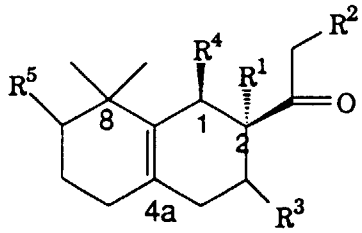

I

zu.

Die Verbindungen I sind durch Cyclisierung der Verbindungen

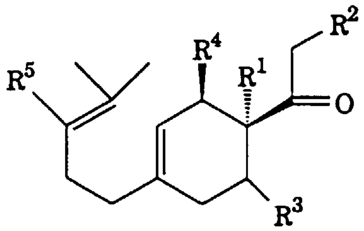

II

unter sauren Bedingungen zugänglich.

Der zweckmässige Zugang zu der Verbindung II kann anhand der Verbindung des untenstehenden Beispiels 1 ausgehend aus Geranial über Homogeraniol, Homomyrcen, und dann durch dessen Diels-Alderreaktion mit einer dienophilen Carbonyl-verbindung, im speziellen, mit Isopropenylmethyl-keton wie folgt veranschaulicht werden:

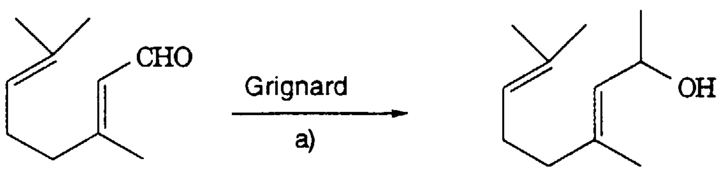


(Geranial) V'

(Homogeraniol) IV'

IV'

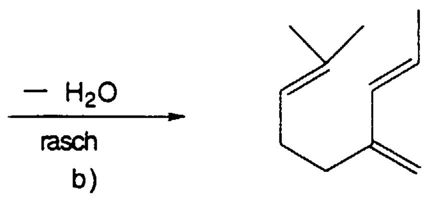


(Methylmyrcen) III'

III'

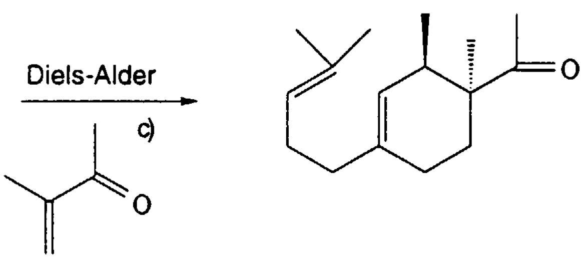


II'

II'

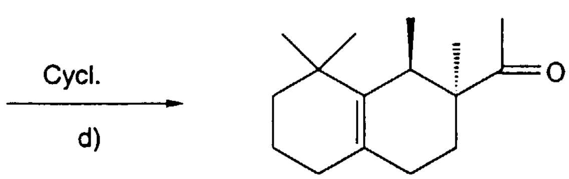


+ Isomer I'

Die geeigneten Reaktionsbedingungen sind dabei:

a) Die Grignard-reaktion mittels $CH_3MgX$ kann in dem Fachmann bekannter Weise erfolgen, also X = Cl, Br, J und in den üblichen inerten Lösungsmitteln, z.B. Tetrahydrofuran, etc., und bei den hier üblichen Temperaturen.

b) Die Wasserabspaltung erfolgt zweckmässigerweise Jod-katalysiert, wobei die Jodkristalle in IV' gelöst werden, und dieses Reagens in kleinen Portionen bei erhöhter Temperatur, nämlich zu einem auf ca. 80° bis ca. 120°C erhitzten Kohlenwasserstoff, z.B. Paraffinöl, zugetropft wird, und das gebildete III' fortwährend abdestilliert wird.

c) Die Diels-Alder Reaktion kann in der dem Fachmann wohlbekannten Weise, also unter dem Einfluss der üblichen Lewissäuren wie z.B. $AlCl_3$, $SnCl_2$, $TiCl_4$, $BF_3$, $BF_3$-Komplexe etc., erfolgen. Geeignete Lösungsmittel sind Aromaten oder Aliphaten, wie Benzol, Toluol, Cyclohexan, Methylcyclohexan, chlorierte Kohlenwasserstoffe wie Chloroform, Methylenchlorid, etc. Die Temperatur beträgt zweckmässigerweise ca. -20°C bis ca. + 50°C.

d) Die Cyclisierung erfolgt zweckmässigerweise unter stark sauren Bedingungen, wobei Mineralsäuren oder starke organische Säuren in Frage kommen. Im Vordergrund steht $H_3PO_4$, insbesondere kristallisierte Phosphorsäure. Lösungsmittel sind insbesondere die oben genannten. Die Temperatur liegt zweckmässigerweise in einem Bereich von ca. 100°C bis ca. 120°C.

Verschiedene aehnliche Reaktionsabläufe sind zwar aus M. Mousseron-Canet et al., Bull. Soc. Chim. France 601 seq. (1959) bekannt geworden.

Die Struktur der Endprodukte wird dort zwar auf Seite 603, Kolonne 2 mit

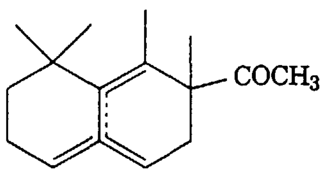

VIII

bezeichnet.

Es hat sich aber herausgestellt, dass bei Nacharbeitung praktisch ausschliesslich die untenstehende Verbindung der Formel

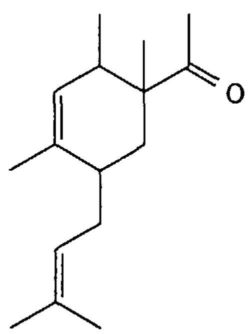

IX

, das 1,2,4-Trimethyl-1-acetyl-5-isopentenyl-cyclohex-3-en anfällt, auf die die recht vagen Bezeichnungen "gem-dimethyl-1-methyl-8-methyl-1-ethylone octaline-9", Seite 606, Kolonne 1 (Stufe d) bzw. Isohexyl-4-methyl-2-methyl-1-ethylone-cyclohexene-4, (Stufe c) überhaupt nicht zutrifft.

Der Grund liegt darin, dass

a) im bekannten Fall ausgehend aus Citral, Seite 600, Kolonne 1, also einem Gemisch von Geranial und Neral ausgegangen wird,

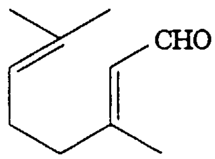

(Geranial)

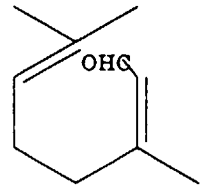

(Neral)

dass b) bei der Wasserabspaltung aus u.a. dem Homogeraniol (IV', Seite 600, Kolonne 1) nicht das gewünschte Honomyrcen, III', Seite 600, Kolonne 2, sondern hauptsächlich das isomere Homoocimen

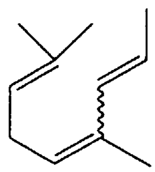

X

vorlag, welches Produkt X in den Stufen c) und d) zu der geruchlich absolut uninteressanten, monocyclischen Verbindung

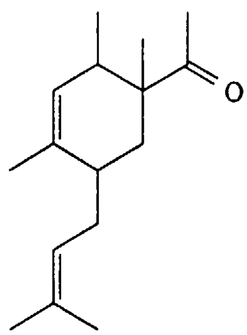

IX

führt.

Diese Folgerungen ergaben sich aus den Resultaten einer genauen Nacharbeitung der entsprechenden, oben identifizierten Stufen von Mousseron et al.

Was schliesslich die von Mousseron et al. auf Seite 603, Kolonne 2, Zeile 30 generisch angesprochenen riechenden Derivate betrifft, muss aufgrund der Resultate der Nacharbeitung angenommen werden, dass hier eben Homoocimenderivate, also monocyclische Derivate der Verbindung X involviert sein mussten.

Die Erfindung betrifft, wie oben gesagt, auch die Verwendung der Verbindungen I als Riechstoffe.

Die Verbindungen I zeichnen sich durch kräftige, diffusive und sehr natürlich-warme Noten Richtung Holz und Ambra aus.

Die Verbindungen der Formel I eignen sich aufgrund ihrer natürlichen Geruchsnoten insbesondere zur Modifizierung von bekannten Kompositionen. Hervorgehoben werden soll insbesondere ihre ausserordentliche Geruchsstärke: Der Geruchsschwellenwert von I' beträgt ~0,1 ng/l; der Geruchswert beträgt 150,000. Bezüglich Definition von Geruchswert und Geruchsschwellenwert siehe z.B. Ulrich A. Huber, Seifen - Oele - Fette - I' Wachse 110, Nr. 15(1984) 448-451.

Die Verbindungen I verbinden sich mit zahlreichen bekannten Riechstoffingredientien natürlichen oder synthetischen Ursprungs, wobei die Palette der natürlichen Rohstoffe sowohl leicht- als auch mittel- und schwer-flüchtige Komponenten, und diejenige der Synthetika Vertreter aus praktisch allen Stoffklassen umfassen kann, wie dies aus der folgenden Zusammenstellung ersichtlich ist:

- Naturprodukte, wie Baummoss-Absolue, Basilikumöl, Agrumenöle (wie Bergamotteöl, Mandarinenöl, etc.), Mastix-Absolue, Myrtenöl, Palmarosaöl, Patchouliöl, Petitgrainöl Paraguay, Wermutöl,

- Alkohole, wie Farnesol, Geraniol, Linalool, Nerol, Phenyläthylalkohol, Rhodinol, Zimtalkohol,

- Aldehyde, wie Citral, Helional®, α-Hexylzimtaldehyd, Hydroxycitronellal, Lilial® (p-tert.Butyl-α-methyl-dihydrozimtaldehyd), Methylnonylacetaldehyd,

- Ketone, wie Allyljonon, α-Jonon, β-Jonon, Isoraldein (Isomethyl-α-ionon), Methyljonon,

- Ester, wie Allyl-phenoxyacetat, Benzyl-salicylat, Cinnamylpropionat, Citronellyl-acetat, Citronellyl-äthoxalat (Citronellyl. O - CO -CO. $OC_2H_5$). Decyl-acetat, Dimethylbenzylcarbinyl-acetat, Dimethylbenzylcarbinyl-butyrat, Aethyl-acetoacetat, Aethyl-acetylacetat, Hexenyl-isobutyrat, Linalylacetat, Methyl-dihydrojasmonat, Styrallylacetat, Vetiverylacetat.

- Lactone, wie γ-Undecalacton,

- verschiedene, in der Parfünerie oft benützte Komponenten, wie Ketonmoschus, Indol, p-Menthan-8-thiol-3-on. Methyleugenol.

Bemerkenswert ist ferner die Art und Weise, wie die Verbindungen I die Geruchsnoten bekannter Kompositionen abrunden, harmonisieren und bereichern, ohne aber in unangenehmer Weise zu dominieren. Hervorgehoben sollen insbesondere auch Stärke, Haftfestigkeit, Wärme, Substantivität und Volumen dieser Verbindungen.

Die Verbindungen der Formel I (bzw. deren Gemische) lassen sich in weiten Grenzen einsetzen, die beispielsweise von 0,1 (Detergentien) - 5% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 0,2 und 2%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mündwässer, Desodorantien, Detergentien, Tabak, etc.).

Die Verbindungen I können demgemäss bei der Herstellung von Kompositionen und - wie obige Zusammenstellung zeigt - unter Verwendung einer breiten Palette bekannter Riechstoffe bzw. Riechstoffgemische, verwendet werden. Bei der Herstellung solcher Kompositionen können die oben aufgeführten bekannten Riechstoffe nach (dem Parfümer bekannter) Art und Weise verwendet werden, wie z.B. aus W.A. Poucher, Perfumes, Cosmetics and Soaps 2, 7. Auflage, Chapman und Hall, London, 1974 hervorgehend.

Auch die Verbindungen der Formel II verfügen über Riechstoffeigenschaften, sie können deshalb analog eingesetzt werden.

Beispiel 1

a) 4,8-Dimethyl-2-hydroxy-nona-3,7-dien (IV')

In einem 4 1/2 l Sulfierkolben mit Thermometer, Rührer und Tropftrichter wird unter $N_2$ 1 l t-Butyl-methyläther und 1.05 l 3N-Methylmagnesiumchlorid-Lösung vorgelegt und bei 0°C 456 g Geranial in 500 ml t-Butyl-methyläther zugetropft. Es wird 1 Stunde bei 10°C gerührt und auf 3 kg Eis und 500 g $NH_4Cl$ gegossen und mit 1 l Hexan versetzt. Nach gutem Mischen wird die $H_20$-Phase abgetrennt und 3 x mit 500 ml $NH_4Cl$-Lösung neutral gewaschen, getrocknet und eingedampft.

Schliesslich wird bei 0.5 mm Hg und 40°C getrocknet,wobei 513 g Oel anfallen; Roh-Ausbeute 100 % d. Theorie.

b) 8-Methyl-4-methyliden-nona-2,7-dien (III')

In einem 1 l 2-Halskolben mit Innenthermometer werden 120 g Paraffinöl vorgelegt. Der Kolben wird mit einem Claisenaufsatz, einem Tropftrichter mit langem Innenrohr und einer Vigreux-Kolonne (60 cm Länge) mit gutem Intensivkühler bestückt, und es wird bei 140 - 147°C Innentemperatur und 10 - 16 mm Hg gerührt. Nun wird eine Lösung von $J_2$ in Alkohol IV' so zugetropft, dass die Kolonnenkopftemperatur 75 - 80°C beträgt. Die Zutropfgeschwindigkeit wird auf 350 - 400 ml/h eingestellt und die Innentemperatur auf 145°C gehalten. Die Dauer beträgt 5 1/2 Stunden, wobei immer 200 g Portionen des Alkohols mit 0.4 g Jod unter $N_2$ gemischt werden. Das $KW/H_2O$-Gemisch wird

periodisch gaschromatographisch überprüft (Gehalt von b) zwischen 59 % und 70 %). Das Destillat wird portionenweise abgetrennt, die $H_2O$-Phase separiert (pH 1 - 2), mit 15 %iger NaOH geschüttelt und auf $K_2CO_3$ im Kühlschrank aufbewahrt. Es fallen aus total 2 kg Rohmaterial total 1780 g gelbes Oel an, Gehalt an b) 61.8%.

Diese 1780 g werden über eine Sulzer-Kolonne von 70 cm am HV unter Zusatz von 20 g $K_2CO_3$ destilliert und so das Methylmyrcen von Rückstand (den anderen Kohlenwasserstoffen) getrennt.

Totalausbeute : 901 g Methylmyrcen (III') (90 %ig) vom Sdp. 40-42°C/0,12 mmHg (45 % der theoretischen Ausbeute über 2 Stufen). Das Produkt wird mit 0.5 % Hydrochinon stabilisiert.

c) 1-Acetyl-1,2-dimethyl-4-(4-methyl-pent-3-enyl)cyclohex-3-en (II')

40 g $AlCl_3$ werden unter $N_2$ in einem 2 1/2 l Sulfierkolben in 1.3 l Methylenchlorid vorgelegt. Unter guter Kühlung wird ein Gemisch von 280 g Methylmyrcen (III') und 134.4 g Isopropenyl-methylketon in 300 Methylenchlorid bei ≤ -15°C zugetropft (Dauer 1 Stde 20 Min). [Alternativ kann in ein Lösung von $AlCl_3$ und Isopropenylmethylketon das Dien getropft werden.] Es wird 30 Minuten nachgerührt und auf 1 1/2 l Eiswasser-Gemisch hydrolisiert. Mit $H_2O$ wird 2 mal neutral gewaschen, mit $K_2CO_3$-Lösung (5 %ig) werden letzte Säurespuren entfernt und nach dem Trocknen wird eingedampft. Es fallen 430 g gelbes Oel an.

Diese 430 g werden am HV über eine 15 cm Vigreux-Kolonne destilliert, wobei 319 g farbloses Oel vom Sdp. 98-114°C/0,2 Torr anfallen (85% der Theorie).

d) (1R*,2S*)-2-Acetyl-1,2,3,4,5,6,7,8-octahydro-1,2,8,8-tetramethylnaphthalin I' [und (2S*,3R*)-2-Acetyl-1,2,3,4,5,6,7,8-Octahydro-2,3,8,8-tetramethylnaphtalin]

In einem 1 1/2 l Sulfierkolben mit Thermometer, Rückflusskühler und Rührer werden unter $N_2$ Begasung 180 g Phosphorsäure vorgelegt und eine Lösung von 310 g Keton II' in 500 ml Toluol zugegeben. Unter Rühren wird auf Rückfluss (115°C) erkühlt und 30 Min. bei dieser Temperatur gerührt. Dann wird schnell abgekühlt und auf 600 ml Eis/ $H_2O$ gegossen, mit 1 l Hexan verdünnt und mit $H_2O$ 2 x ausgeschüttet, mit 10 %iger NaOH gewaschen, getrocknet und eingedampft. Es resultieren 325 g Oel,

Destilliert am HV, ergeben sich 224,6 g Oel vom Sdp. 92-96°C/0,09 mmHg.

Ausbeute an olfaktisch gutem Produkt 224.6 g (74.2 % der Theorie).

Geruch : ambrig, holzig, Tabak.

Beispiel 2

Auf analoge Weise wird aus Homomyrcen (III') und 2-Methyl-2-cyclopenten-1-on über das (3aRS,7SR,7aRS)-5-(4-methyl-pent-3-enyl)-7,7a-dimethyl-2,3,3a,4,7,7a-hexahydro-1H-inden-1-on das (3aRS,9SR,9aRS)-8,8,9,9a-tetramethyl-2,3,3a,4,5,6,7,8,9,9a-decahydro-1H-benz<f>inden-1-on erhalten.

Geruch: ambrig, holzig.

Beispiel 3

Ebenfalls analog kann aus Homomyrcen (III') und 2-Methyliden-1-cyclopentanon über das entsprechende Spirocyclopentanonderivat II rac-1',2',3',4',5',6', 7', 8'-Octahydro-1'alpha,8',8'-trimethyl-spiro[cyclopentan-(alpha2),2'(1'H)-naphthalin-]-2-on-erhalten werden.

Geruch: holzig, ambrig, nach Sclareol.

Beispiel 4

Schliesslich ist aus Homomyrcen (III') und Methyl-vinylketon über das 1-Acetyl-2-methyl-4-(4-methyl-pent-3-enyl)-cyclohex-3-en das 2-Acetyl-1,2,3,4,5,6,7,8-octahydro-1,8,8-trimethylnaphthalin zugänglich.

Geruch: trocken, holzig, ambrig.

Beispiel 5

Aus Aethylmyrcen (Bishomomyrcen) wird in Analogie zu Beispiel 1 das rac-2α-Acetyl-1α-ethyl-1,2,3,4,5,6,7,8-octahydro-2,8,8-trimethylnaphthalin erhalten.

Geruch: holzig, ambrig.

Beispiel 6

Ebenfalls analog Beispiel 1, aber durch Reaktion von Methylmyrcen mit Isopropenyl-äthylketon wird das rac-2α-Propionyl-1,2,3,4,5,6,7,8-octahydro-1α,2,8,8-tetramethylnaphthalin erhalten.

Geruch: holzig, ambrig.

Beispiel 7

a) Eau de Toilette pour femmes

| | Gewichtsteile |
|---|---|
| Cyclohexal | 54.0000 |
| Dipropylen-glycol | 348.2000 |
| Galaxolide 50 BB (1,3,4,6,7,8-Hexahydro-4,6,6,7,8,8-hexamethyl-cyclopenta-γ-2-benzopyran) | 262.0000 |
| Verbindung I' | 37.0000 |
| Hedione (Methyldihydrojasmonat) | 55.0000 |
| Heliotropin crist. | 11.0000 |
| Isoraldein 95 (Isomethyl-α-ionon) | 206.0000 |
| Linalool synt. | 1.0000 |
| Pêche pure (γ-Undecalacton) | 1.8000 |
| Radjanol (Bagdanol) (1-β-Hydroxymethyl-2-pentenyl)-2,2,3-trimethyl-cyclopent-3-en) | 8.0000 |
| Vanillin | 16.0000 |
| | 1000.0000 |

b) Eau de Toilette pour hommes

| | Gewichtsteile |
|---|---|
| Phenyl-ethyl-alkohol | 2.0000 |
| Allyl-amyl-glycolat | 18.0000 |
| Ambroxan (3-Methyldodecahydro-6,6,9A-trimethyl-naphth-2,1B-furan) | 3.0000 |
| Bergamotte-essenz | 130.0000 |
| Carbitol (Diethylenglykol-monoethylether) | 20.0000 |
| Citronen-essenz | 20.0000 |
| Citronellol | 3.0000 |
| Cyclohexal | 10.0000 |
| α-Damascon | 4.0000 |
| Dihydro-myrcenol | 150.0000 |
| Evernyl (Methyl-β-orcincarboxylat) | 5.0000 |
| Fixolide (1,1,2,4,4,7-Hexamethyl-6-acetyl-1,2,3,4-tetrahydro-naphthalin) | 70.0000 |
| Galbex 183 (Komposition Richtung Galbanum) | 3.0000 |
| Geranium-essenz | 5.0000 |
| Verbindung I' | 7.0000 |
| Hedione | 55.0000 |
| Fixateur Hercolyn (Dihydro/Tetrahydro-methylabietat) | 25.0000 |
| Hydroxycitronellal | 20.0000 |
| Isoraldein 40 | 5.0000 |
| Lavandin | 50.0000 |
| Lemarome N (Neral-Citralgemisch) | 3.0000 |
| Mandarinen-essenz | 10.0000 |
| Methyl-cedryl-keton | 80.0000 |

(fortgesetzt)

| | Gewichtsteile |
|---|---|
| Oranger crist. (γ-Undecalacton) | 2.0000 |
| Diethyl-phthalat | 267.0000 |
| Precyclemone B (Dehydrovernaldehyd) | 10.0000 |
| Rosmarin-essenz | 10.0000 |
| Rosoflor 2 (Geraniol-nerol-gemisch) | 3.0000 |
| Amyl-salicylat | 5.0000 |
| Sandalore (3-Methyl-5-(2,2,3-trimethyl-cyclopent-3-en-1-yl)-pentan-2-ol) | 5.0000 |
| | 1000.0000 |

c) Blumig-holziger, leicht würziger Aspekt

| | Gewichtsteile |
|---|---|
| Benzyl-acetat extra | 40.0000 |
| Dimethyl-benzyl-carbinyl-acetat | 40.0000 |
| Geranyl-acetat synt. | 30.0000 |
| Phenyl-ethyl-alkohol | 100.0000 |
| Bergamotte-öl | 100.0000 |
| Cetone-α (α-Methylionon) | 80.0000 |
| Cyclohexal | 40.0000 |
| Dipropylen-glycol | 80.0000 |
| Eugenol | 20.0000 |
| Galaxolide 50 DEP | 40.0000 |
| Gardenol (Phenyl-methyl-carbinylacetat) | 10.0000 |
| Geraniol | 50.0000 |
| Verbindung I' | 20.0000 |
| Hedione | 40.0000 |
| Heliotropin crist. | 20.0000 |
| Hydroxycitronellal | 50.0000 |
| Methyl-cedryl-keton | 60.0000 |
| Methyl-isoeugenol | 10.0000 |
| Moschusketon | 50.0000 |
| Benzyl-salicylat | 100.0000 |
| Thibetolide (Ω-Pentadecalactone) | 20.0000 |
| | 1000.0000 |

**Patentansprüche**

**1.** Verbindungen der Formel

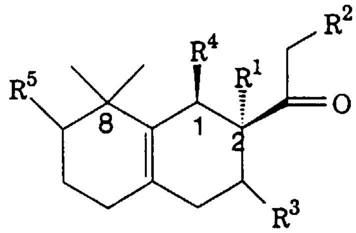

I

worin

$$R^1 = R^4 = CH_3 \text{ und } R^2 = R^3 = H$$

oder

$$R^1 = R^2 = R^3 = H \text{ und } R^4 = CH_3,$$

oder

$$R^1 + R^2 = -CH_2CH_2-$$

und

$$R^3 = H \text{ und } R^4 = CH_3,$$

oder

$$R^1 = R^4 = CH_3 \text{ und } R^2 + R^3 = -CH_2-$$

oder

$$R^1 = CH_3 \text{ und } R^2 = R^3 = H$$

und

$$R^4 = CH_2CH_3$$

oder

$$R^1 = R^2 = R^4 = CH_3$$

und

$$R^3 = H$$

sind, und

$$R^5 = H$$

(bevorzugt) oder $CH_3$ ist.

**2.** Verbindungen der Formel

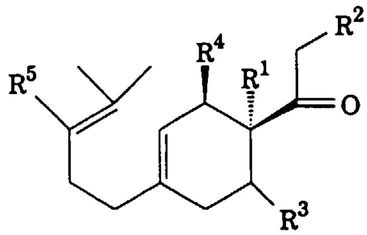

II

worin

$$R^1 = R^4 = CH_3 \text{ und } R^2 = R^3 = H$$

oder

$$R^1 = R^2 = R^3 = H \text{ und } R^4 = CH_3,$$

oder

$$R^1 + R^2 = -CH_2CH_2-$$

und

$$R^3 = H \text{ und } R^4 = CH_3,$$

oder

$$R^1 = R^4 = CH_3 \text{ und } R^2 + R^3 = -CH_2-$$

oder

$$R^1 = CH_3 \text{ und } R^2 = R^3 = H$$

und

$$R^4 = CH_2CH_3$$

oder

$$R^1 = R^2 = R^4 = CH_3$$

und

$$R^3 = H$$

sind, und

$R^5 = H$

(bevorzugt) oder $CH_3$ ist.

**3.** Verfahren zur Herstellung der Verbindungen

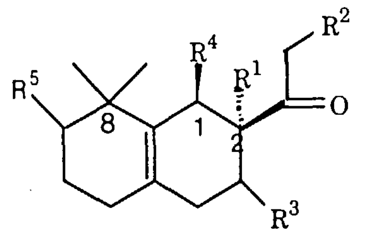

I

worin

$R^1 = R^4 = CH_3$ und $R^2 = R^3 = H$

oder

$R^1 = R^2 = R^3 = H$ und $R^4 = CH_3$,

oder

$R^1 + R^2 = -CH_2CH_2-$

und

$R^3 = H$ und $R^4 = CH_3$,

oder

$R^1 = R^4 = CH_3$ und $R^2 + R^3 = -CH_2-$

oder

$R^1 = CH_3$ und $R^2 = R^3 = H$

und

$R^4 = CH_2CH_3$

oder

$R^1 = R^2 = R^4 = CH_3$

und

$$R^3 = H$$

sind, und

$$R^5 = H$$

(bevorzugt) oder $CH_3$ ist, dadurch gekennzeichnet, dass man eine Verbindung der Formel

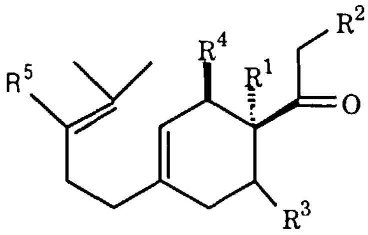

II

,worin die Substituenten $R^1$-$R^5$ ebenfalls die vorgenannte Bedeutung haben, unter sauren Bedingungen cyclisiert.

**4.** Riechstoffkomposition, gekennzeichnet durch einen Gehalt an einer Verbindung der Formel

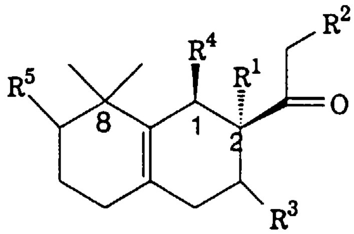

I

worin

$$R^1 = R^4 = CH_3 \text{ und } R^2 = R^3 = H$$

oder

$$R^1 = R^2 = R^3 = H \text{ und } R^4 = CH_3,$$

oder

$$R^1 + R^2 = -CH_2CH_2-$$

und

$$R^3 = H \text{ und } R^4 = CH_3,$$

oder

$$R^1 = R^4 = CH_3 \text{ und } R^2 + R^3 = -CH_2-$$

oder

$$R^1 = CH_3 \text{ und } R^2 = R^3 = H$$

und

$$R^4 = CH_2CH_3$$

oder

$$R^1 = R^2 = R^4 = CH_3$$

und

$$R^3 = H$$

sind, und

$$R^5 = H$$

(bevorzugt) oder $CH_3$ ist.

**5.** Verwendung der Verbindungen der Formel

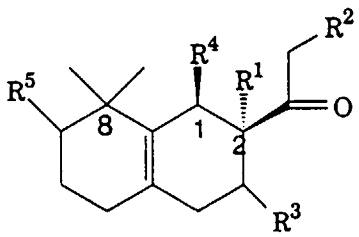

I

worin

$$R^1 = R^4 = CH_3 \text{ und } R^2 = R^3 = H$$

oder

$$R^1 = R^2 = R^3 = H \text{ und } R^4 = CH_3,$$

oder

$$R^1 + R^2 = -CH_2CH_2-$$

und

$$R^3 = H \text{ und } R^4 = CH_3,$$

oder

$$R^1 = R^4 = CH_3 \text{ und } R^2 + R^3 = \text{-}CH_2\text{-}$$

oder

$$R^1 = CH_3 \text{ und } R^2 = R^3 = H$$

und

$$R^4 = CH_2CH_3$$

oder

$$R^1 = R^2 = R^4 = CH_3$$

und

$$R^3 = H$$

sind, und

$$R^5 = H$$

(bevorzugt) oder $CH_3$ ist,
als Riechstoffe.

**6.** Riechstoffkomposition, gekennzeichnet durch einen Gehalt an einer Verbindung der Formel

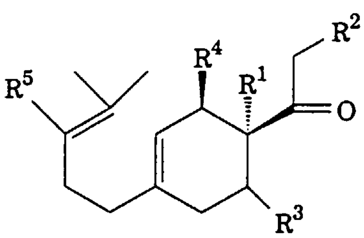

II

worin

$$R^1 = R^4 = CH_3 \text{ und } R^2 = R^3 = H$$

oder

$$R^1 = R^2 = R^3 = H \text{ und } R^4 = CH_3,$$

oder

$$R^1 + R^2 = \text{-}CH_2CH_2\text{-}$$

und

$$R^3 = H \text{ und } R^4 = CH_3,$$

oder

$$R^1 = R^4 = CH_3 \text{ und } R^2 + R^3 = -CH_2-$$

oder

$$R^1 = CH_3 \text{ und } R^2 = R^3 = H$$

und

$$R^4 = CH_2CH_3$$

oder

$$R^1 = R^2 = R^4 = CH_3$$

und

$$R^3 = H$$

sind, und

$$R^5 = H$$

(bevorzugt) oder $CH_3$ ist.

**7.** Verwendung der Verbindungen der Formel

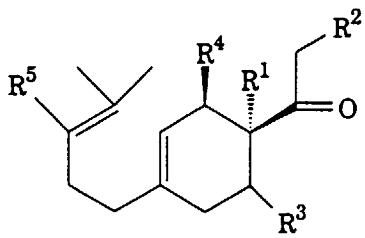

II

worin

$$R^1 = R^4 = CH_3 \text{ und } R^2 = R^3 = H$$

oder

$$R^1 = R^2 = R^3 = H \text{ und } R^4 = CH_3,$$

oder

$$R^1 + R^2 = -CH_2CH_2-$$

und

$$R^3 = H \text{ und } R^4 = CH_3,$$

oder

$$R^1 = R^4 = CH_3 \text{ und } R^2 + R^3 = -CH_2-$$

oder

$$R^1 = CH_3 \text{ und } R^2 = R^3 = H$$

und

$$R^4 = CH_2CH_3$$

oder

$$R^1 = R^2 = R^4 = CH_3$$

und

$$R^3 = H$$

sind, und

$$R^5 = H$$

(bevorzugt) oder $CH_3$ ist
als Riechstoffe.

## Claims

**1.** Compounds having the formula

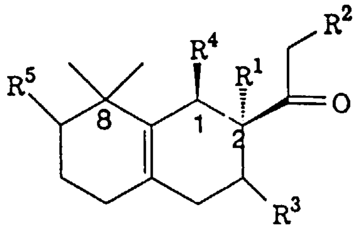

I

wherein

$R^1 = R^4 = CH_3$ and $R^2 = R^3 = H$

or

$R^1 = R^2 = R^3 = H$ and $R^4 = CH_3$,

or

$R^1 + R^2 = -CH_2CH_2-$

and

$R^3 = H$ and $R^4 = CH_3$

or

$R^1 = R^4 = CH_3$ and $R^2 + R^3 = -CH_2-$

or

$R^1 = CH_3$ and $R^2 = R^3 = H$

and

$R^4 = CH_2CH_3$

or

$R^1 = R^2 = R^4 = CH_3$

and

$R^3 = H$,

and

$R^5 = H$

(preferably) or $CH_3$.

**2.** Compounds having the formula

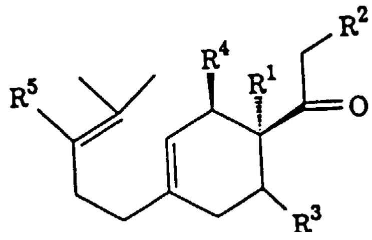

II

wherein

$$R^1 = R^4 = CH_3 \text{ and } R^2 = R^3 = H$$

or

$$R^1 = R^2 = R^3 = H \text{ and } R^4 = CH_3,$$

or

$$R^1 + R^2 = -CH_2CH_2-$$

and

$$R^3 = H \text{ and } R^4 = CH_3$$

or

$$R^1 = R^4 = CH_3 \text{ and } R^2 + R^3 = -CH_2-$$

or

$$R^1 = CH_3 \text{ and } R^2 = R^3 = H$$

and

$$R^4 = CH_2CH_3$$

or

$$R^1 = R^2 = R^4 = CH_3$$

and

$$R^3 = H,$$

and

$$R^5 = H$$

(preferably) or $CH_3$.

**3.** A process for preparation of the compounds

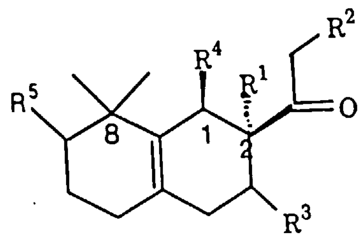

I

wherein

$$R^1 = R^4 = CH_3 \text{ and } R^2 = R^3 = H$$

or

$$R^1 = R^2 = R^3 = H \text{ and } R^4 = CH_3,$$

or

$$R^1 + R^2 = -CH_2CH_2-$$

and

$$R^3 = H \text{ and } R^4 = CH_3$$

or

$$R^1 = R^4 = CH_3 \text{ and } R^2 + R^3 = -CH_2-$$

or

$$R^1 = CH_3 \text{ and } R^2 = R^3 = H$$

and

$$R^4 = CH_2CH_3$$

or

$$R^1 = R^2 = R^4 = CH_3$$

and

$$R^3 = H,$$

and

$$R^5 = H$$

(preferably) or $CH_3$, characterised in that a compound having the formula

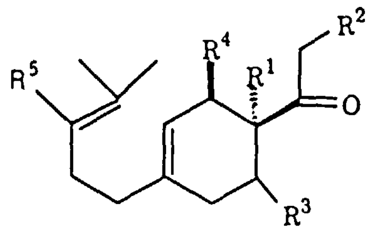

II

wherein the substituents $R^1$-$R^5$ also have the meaning given previously, is cyclised under acid conditions.

**4.** A perfume composition characterised in that it contains a compound havina the formula

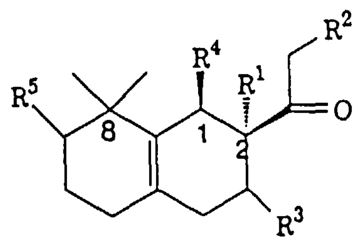

I

wherein

$$R^1 = R^4 = CH_3 \text{ and } R^2 = R^3 = H$$

or

$$R^1 = R^2 = R^3 = H \text{ and } R^4 = CH_3,$$

or

$$R^1 + R^2 = -CH_2CH_2-$$

and

$$R^3 = H \text{ and } R^4 = CH_3$$

or

$$R^1 = R^4 = CH_3 \text{ and } R^2 + R^3 = -CH_2-$$

or

$$R^1 = CH_3 \text{ and } R^2 = R^3 = H$$

and

$$R^4 = CH_2CH_3$$

or

$$R^1 = R^2 = R^4 = CH_3$$

and

$$R^3 = H,$$

and

$$R^5 = H$$

(preferably) or $CH_3$.

**5.** Use of the compounds having the formula

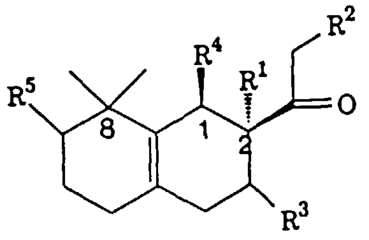

I

wherein

$$R^1 = R^4 = CH_3 \text{ and } R^2 = R^3 = H$$

or

$$R^1 = R^2 = R^3 = H \text{ and } R^4 = CH_3,$$

or

$$R^1 + R^2 = -CH_2CH_2-$$

and

$$R^3 = H \text{ and } R^4 = CH_3$$

or

$$R^1 = R^4 = CH_3 \text{ and } R^2 + R^3 = -CH_2-$$

or

$$R^1 = CH_3 \text{ and } R^2 = R^3 = H$$

and

$$R^4 = CH_2CH_3$$

or

$$R^1 = R^2 = R^4 = CH_3$$

and

$$R^3 = H,$$

and

$$R^5 = H$$

(preferably) or $CH_3$, as perfumes.

**6.** A perfume composition characterised in that it contains a compound having the formula

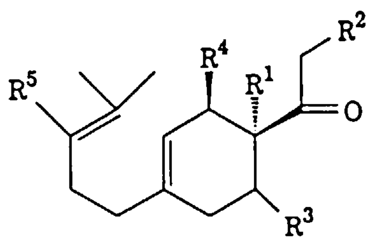

II

wherein

$$R^1 = R^4 = CH_3 \text{ and } R^2 = R^3 = H$$

or

$$R^1 = R^2 = R^3 = H \text{ and } R^4 = CH_3,$$

or

$$R^1 + R^2 = -CH_2CH_2-$$

and

$$R^3 = H \text{ and } R^4 = CH_3$$

or

$$R^1 = R^4 = CH_3 \text{ and } R^2 + R^3 = -CH_2-$$

or

$$R^1 = CH_3 \text{ and } R^2 = R^3 = H$$

and

$$R^4 = CH_2CH_3$$

or

$$R^1 = R^2 = R^4 = CH_3$$

and

$$R^3 = H,$$

and

$$R^5 = H$$

(preferably) or $CH_3$.

**7.** Use of the compounds having the formula

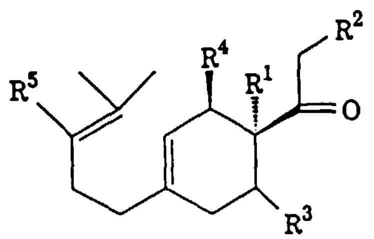

II

wherein

$$R^1 = R^4 = CH_3 \text{ and } R^2 = R^3 = H$$

or

$$R^1 = R^2 = R^3 = H \text{ and } R^4 = CH_3,$$

or

$$R^1 + R^2 = -CH_2CH_2-$$

and

$$R^3 = H \text{ and } R^4 = CH_3$$

or

$$R^1 = R^4 = CH_3 \text{ and } R^2 + R^3 = -CH_2-$$

or

$$R^1 = CH_3 \text{ and } R^2 = R^3 = H$$

and

$$R^4 = CH_2CH_3$$

or

$$R^1 = R^2 = R^4 = CH_3$$

and

$$R^3 = H,$$

and

$$R^5 = H$$

(preferably) or $CH_3$, as perfumes.

**Revendications**

**1.** Composés de formule

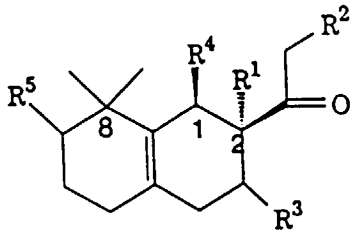

I

dans laquelle

$$R^1 = R^4 = CH_3 \text{ et } R^2 = R^3 = H$$

ou

$R^1 = R^2 = R^3 = H$ et $R^4 = CH_3$

ou

$R^1 + R^2 = -CH_2CH_2-$

et

$R^3 = H$ et $R^4 = CH_3$

ou

$R^1 = R^4 = CH_3$ et $R^2 = R^3 = -CH_2-$

ou

$R^1 = CH_3$ et $R^2 = R^3 = H$

et

$R^4 = CH_2CH_3$

ou

$R^1 = R^2 = R^4 = CH_3$

et

$R^3 = H$,

et

$R^5 = H$

(de préférence) ou $CH_3$.

**2.** Composés de formule

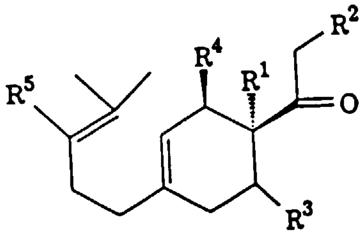

II

dans laquelle

$$R^1 = R^4 = CH_3 \text{ et } R^2 = R^3 = H$$

ou

$$R^1 = R^2 = R^3 = H \text{ et } R^4 = CH_3$$

ou

$$R^1 + R^2 = -CH_2CH_2-$$

et

$$R^3 = H \text{ et } R^4 = CH_3$$

ou

$$R^1 = R^4 = CH_3 \text{ et } R^2 = R^3 = -CH_2-$$

ou

$$R^1 = CH_3 \text{ et } R^2 = R^3 = H$$

et

$$R^4 = CH_2CH_3$$

ou

$$R^1 = R^2 = R^4 = CH_3$$

et

$$R^3 = H,$$

et

$$R^5 = H$$

(de préférence) ou $CH_3$.

**3.** Procédé de préparation des composés de formule

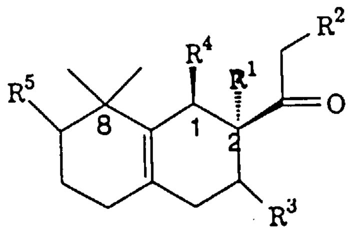

I

dans laquelle

$$R^1 = R^4 = CH_3 \text{ et } R^2 = R^3 = H$$

ou

$$R^1 = R^2 = R^3 = H \text{ et } R^4 = CH_3$$

ou

$$R^1 + R^2 = -CH_2CH_2-$$

et

$$R^3 = H \text{ et } R^4 = CH_3$$

ou

$$R^1 = R^4 = CH_3 \text{ et } R^2 = R^3 = -CH_2-$$

ou

$$R^1 = CH_3 \text{ et } R^2 = R^3 = H$$

et

$$R^4 = CH_2CH_3$$

ou

$$R^1 = R^2 = R^4 = CH_3$$

et

$$R^3 = H,$$

et

$$R^5 = H$$

(de préférence) ou $CH_3$,
caractérisé en ce qu'on cyclise dans des conditions acides un composé de formule

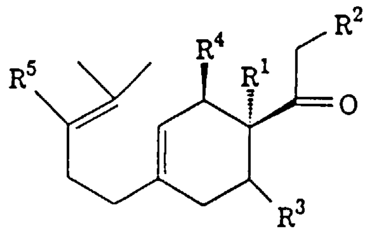

II

dans laquelle les substituants $R^1$-$R^5$ ont la signification donnée ci-dessus.

**4.** Composition de parfum, caractérisée en ce qu'elle contient un composé de formule

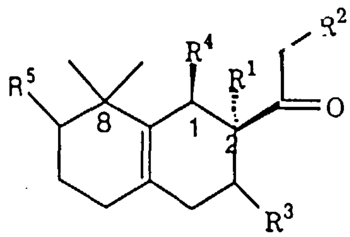

I

dans laquelle

$$R^1 = R^4 = CH_3 \text{ et } R^2 = R^3 = H$$

ou

$$R^1 = R^2 = R^3 = H \text{ et } R^4 = CH_3$$

ou

$$R^1 + R^2 = -CH_2CH_2-$$

et

$$R^3 = H \text{ et } R^4 = CH_3$$

ou

$$R^1 = R^4 = CH_3 \text{ et } R^2 + R^3 = -CH_2-$$

ou

$R^1 = CH_3$ et $R^2 = R^3 = H$

et

$R^4 = CH_2CH_3$

ou

$R^1 = R^2 = R^4 = CH_3$

et

$R^3 = H$,

et

$R^5 = H$

(de préférence) ou $CH_3$.

**5.** Utilisation des composés de formule

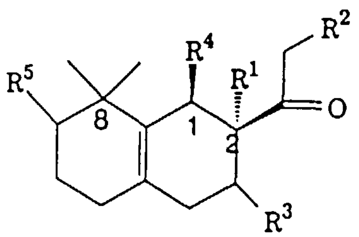

I

dans laquelle

$R^1 = R^4 = CH_3$ et $R^2 = R^3 = H$

ou

$R^1 = R^2 = R^3 = H$ et $R^4 = CH_3$

ou

$R^1 + R^2 = -CH_2CH_2-$

et

$R^3 = H$ et $R^4 = CH_3$

ou

$R^1 = R^4 = CH_3$ et $R^2 + R^3 = -CH_2-$

ou

$R^1 = CH_3$ et $R^2 = R^3 = H$

et

$R^4 = CH_2CH_3$

ou

$R^1 = R^2 = R^4 = CH_3$

et

$R^3 = H$,

et

$R^5 = H$

(de préférence) ou $CH_3$,
comme parfums.

**6.** Composition de parfum, caractérisée en ce qu'elle contient un composé de formule

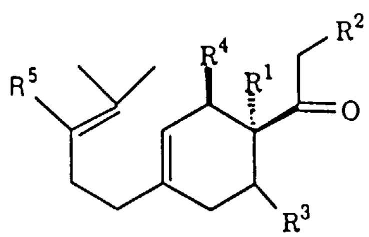

II

dans laquelle

$R^1 = R^4 = CH_3$ et $R^2 = R^3 = H$

ou

$R^1 = R^2 = R^3 = H$ et $R^4 = CH_3$

ou

$R^1 + R^2 = -CH_2CH_2-$

et

$$R^3 = H \text{ et } R^4 = CH_3$$

ou

$$R^1 = R^4 = CH_3 \text{ et } R^2 = R^3 = -CH_2-$$

ou

$$R^1 = CH_3 \text{ et } R^2 = R^3 = H$$

et

$$R^4 = CH_2CH_3$$

ou

$$R^1 = R^2 = R^4 = CH_3$$

et

$$R^3 = H,$$

et

$$R^5 = H$$

(de préférence) ou $CH_3$.

**7.** Utilisation des composés de formule

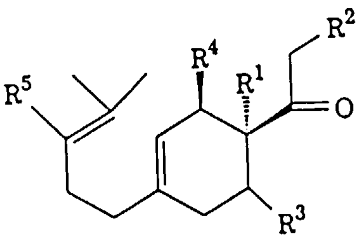

II

dans laquelle

$$R^1 = R^4 = CH_3 \text{ et } R^2 = R^3 = H$$

ou

$$R^1 = R^2 = R^3 = H \text{ et } R^4 = CH_3$$

ou

$$R^1 + R^2 = -CH_2CH_2-$$

et

$$R^3 = H \text{ et } R^4 = CH_3$$

ou

$$R^1 = R^4 = CH_3 \text{ et } R^2 + R^3 = -CH_2-$$

ou

$$R^1 = CH_3 \text{ et } R^2 = R^3 = H$$

et

$$R^4 = CH_2CH_3$$

ou

$$R^1 = R^2 = R^4 = CH_3$$

et

$$R^3 = H,$$

et

$$R^5 = H$$

(de préférence) ou $CH_3$ comme parfums.